# EUROPÄISCHE PATENTSCHRIFT

(11) **EP 0 942 712 B1**
(45) Veröffentlichungstag und Bekanntmachung des Hinweises auf die Patenterteilung: **26.03.2003**
(21) Anmeldenummer: 97953707.3
(22) Anmeldetag: 27.11.1997
(51) Int. Cl.: A61K 7/42

(54) **KOSMETISCHE ZUBEREITUNGEN**
COSMETIC PREPARATIONS
PREPARATIONS COSMETIQUES

(30) Priorität: 05.12.1996 DE 19650473
(43) Veröffentlichungstag der Anmeldung: 22.09.1999
(73) Patentinhaber: Cognis Deutschland GmbH & Co. KG, 40589 Düsseldorf (DE)
(72) Erfinder: WADLE, Armin, D - 40699 Erkrath (DE)
(86) Internationale Anmeldenummer: EP9706613
(87) Internationale Veröffentlichungsnummer: WO98024406

(56) Entgegenhaltungen:
- EP-A- 0 715 845
- DE-A- 19 643 062
- DE-U- 29 520 816

## Beschreibung

### Gebiet der Erfindung

Die Erfindung betrifft kosmetische Zubereitungen, vorzugsweise Selbstbräunungsmittel, enthaltend ausgewählte nichtionische Emulgatoren und Dihydroxyaceton sowie die Verwendung dieser Emulgatoren zur Herstellung dieser Zubereitungen.

### Stand der Technik

In der modernen Leistungsgesellschaft wird von vielen die Bräunung der Haut mit Begriffen wie "jung" und "dynamisch" assoziiert. Für Verbraucher, die sich weder natürlicher noch künstlicher UV-Bestrahlung aussetzen können oder wollen, aber dennoch eine Hautbräunung wünschen, bietet die kosmetische Industrie Selbstbräunungspräparate an, die als Wirkstoff ganz überwiegend Dihydroxyaceton (DHA) enthält.

Ein Problem beim Einsatz von DHA besteht darin, daß die Stabilität in kosmetischen Produkten viel-fach unzureichend ist und es zu unerwünschten Nebenreaktionen, wie z.B. zur Bildung von Formalde-hyd oder Ameisensäure kommen kann; dies versucht man in der Regel durch Mikroverkapselung zu verhindern **[Euro Cosm. No.11, 26 (1995)].** Aus der Europäischen Patentanmeldung **EP-A1 0689125** (L'Oréal) sind kosmetische O/W-Emulsionen mit DHA bekannt, die eine Teilchengröße im Bereich von 100 bis 1000 nm aufweisen. Gemäß der Europäischen Patentanmeldung **EP-A1 0715845** (L'Oréal) werden Selbstbräunungsmittel mit Dihydroxyaceton erhalten, indem man als Emulgator eine Mischung aus einem Alkylpolyglucosid und einem Fettalkohol einsetzt. Aus der Europäischen Patentanmeldung **EP-A1 0671159** (Shiseido) sind ferner Zusammensetzungen bekannt, die neben DHA, Ölkörper und Ethylenoxid/Propylenoxid-Blockpolymere enthalten. Zu erwähnen bleibt ferner die deutsche Gebrauchsmusteranmeldung **DE 295 20 816 U1**, die kosmetische und/oder pharmazeutische Emulsionen betrifft, die zur Vermeidung hochviskoser lamellarer Gele eine Kombination aus Alkyloligoglucosiden, Fettalkoholen und/oder Polyolpoly-12-hydroxystearaten, sowie Guerbetalkoholen enthält.

Problematisch ist ferner die Tatsache, daß die Zubereitungen in aller Regel bei Raumtemperatur homogen sind und eine konstante Viskosität aufweisen, bei Erwärmung auf beispielsweise 35 bis 40°C jedoch schon nach kurzer Zeit eine irreversible Tendenz zur Entmischung zeigen und ihre Viskosität einbüßen. Wird ein solches Präparat angewendet, kommt es zu einer ungleichen Verteilung der selbstbräunenden Inhaltsstoffe auf der Haut, mit der Folge, daß der Bräunungseffekt unregelmäßig ausfällt. Es ist sofort klar, daß dies vom Verbraucher nicht toleriert werden kann.

Die Aufgabe der Erfindung hat somit darin bestanden, kosmetische Zubereitungen mit einem Gehalt an Dihydroxyaceton zur Verfügung zu stellen, die sich gleichzeitig durch eine hohe dermatologische Verträglichkeit, ein vorteilhaftes Hautgefühl sowie insbesondere eine hohe Lagerstabilität bei höheren Temperaturen auszeichnen. Ein wesentlicher Punkt in der Aufgabenstellung hat also darin bestanden, die Nachteile des Stands der Technik zu überwinden und sowohl die chemische Zersetzung des DHA als auch die ungleichmäßige Verteilung des Dihydroxyacetons bei Temperaturlagerung in den Zubereitungen zuverlässig zu verhindern.

### Beschreibung der Erfindung

Gegenstand der Erfindung sind kosmetische Zubereitungen, enthaltend
(a) Alkyl- und/oder Alkenyloligoglykoside,
(b) Polyolpoly-12-hydroxystearate und
(c) Dihydroxyaceton.

Überraschenderweise wurde gefunden, daß unter Verwendung von Mischungen aus Alkyl- und/oder Alkenyloligoglykosiden und Polyolpoly-12-hydroxystearaten als nichtionische Emulgatoren kosmetische Zubereitungen mit einem Gehalt an Dihydroxyaceton erhalten werden, die sich durch eine ausgezeichnete Lagerstabilität auch bei erhöhten Temperaturen auszeichnen. Auf diese Weise wird sichergestellt, daß die homogenen, viskosen Mittel auch bei direkter Sonneneinstrahlung ihre Stabilität nicht verlieren und vom Verbraucher leicht und zuverlässig angewendet werden können. Gleichzeitig wird eine hohe Stabilität des DHA beobachtet, ohne daß eine Mikroverkapselung erforderlich wäre.

### Alkyl- und/oder Alkenyloligoglykoside

Alkyl- und Alkenyloligoglykoside stellen bekannte nichtionische Tenside dar, die der Formel (I) folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0 301 298** und **WO 90/03977** verwiesen.

Die Alkyl- und/oder Alkenytoligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispieie sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Polyolpoly-12-hydroxystearate

Bei den Polyolpoly-12-hydroxystearaten handelt es sich um bekannte Stoffe, die beispielsweise unter den Marken "Dehymuls® PGPH" oder "Eumulgin® VL 75" (Abmischung mit Coco Glucosides im Gewichtsverhältnis 1:1) von der Henkel KGaA, Düsseldorf/FRG vertrieben werden. In diesem Zusammenhang sei ferner auf die internationale Patentanmeldung **WO 95/34528** (Henkel) verwiesen. Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methyolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit,
(f) Zucker mit 5 bis 12 Kohlenstoffatomen, wie beispielsweise Glucose oder Saccharose;
(g) Aminozucker wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):

| | |
|---|---|
| Glycerin | 5 bis 35 (15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20 (8 bis 15) Gew.-% |
| Pentaglycerine | 2 bis 10 (3 bis 8) Gew.-% |
| Oligoglycerine | ad 100 Gew.-% |

Das Mischungsverhältnis der beiden Komponenten (a) und (b) kann 90 : 10 bis 10 : 90, vorzugsweise 75 : 25 bis 25 : 75 und insbesondere 60 : 40 bis 40 : 60 betragen.

### Ölkörper

Die erfindungsgemäßen Zubereitungen enthalten als weitere Bestandteile vorzugsweise Ölkörper. Hierfür kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alko-holen (z.B. Finsolv® TN), Dialkylether, Siliconöle und/oder aliphatische bzw. naphthenische Kohlenwasserstoffe in Betracht.

In einer bevorzugten Ausführungsform der Erfindung, enthalten die kosmetischen Zubereitungen
(a) 0,1 bis 30, vorzugsweise 1 bis 15 Gew.-% Alkyl- und/oder Alkenyoligoglykoside,
(b) 0,1 bis 30, vorzugsweise 1 bis 15 Gew.-% Polyolpoly-12-hydroxystearate,
(c) 0,01 bis 6, vorzugsweise 0,5 bis 4 Gew.-% Dihydroxyaceton und
(d) 1 bis 90, vorzugsweise 25 bis 75 Gew.-% Ölkörper,
mit der Maßgabe, daß sich die Mengenangaben gegebenenfalls mit Wasser und üblichen Hilfs- und Zusatzstoffen zu 100 Gew.-% ergänzen.

### Gewerbliche Anwendbarkeit

Mit Hilfe der nichtionischen Emulgatormischungen aus Alkyl- und/oder Alkenyloligoglykosiden und Polyolpoly-12-hydroxystearaten werden zusammen mit Dihydroxyaceton auch bei höheren Temperaturen lagerstabile Zubereitungen, vorzugsweise Selbstbräunungsmittel in Form vom O/W-Emulsionen erhalten. Ein weiterer Gegenstand der Erfindung betrifft daher die Verwendung Mischungen aus (a) Alkyl - und/oder Alkenyloligoglykosiden und (b) Polyolpoly-12-hydroxystearaten als nichtionische Emulgatoren für Dihydroxyaceton zur Herstellung von Selbstbräunungsmitteln. In einer bevorzugten Ausführungsform der Erfiondung werden Mischungen eingesetzt, die (a) Alkyloligoglucoside und (b) Polyglycerinpoly-12-hydroxysterate im Gewichtsverhältnis 25 : 75 bis 75 : 25, vorzugsweise 40 : 60 bis 60 : 40 und desweiteren ein Polyol, vorzugsweise Glycerin enthalten. Derartige Mischungen sind unter der Marke "Eumulgin® VL 75" im Handel.

Die erfindungsgemäßen Zubereitungen, wie beispielsweise Haarshampoos, Haarlotionen, Schaumbäder, Cremes, Lotionen oder Salben, können ferner als weitere Hilfs- und Zusatzstoffe milde Tenside, Co-Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, Insektenrepellentien, Farb- und Duftstoffe enthalten.

Typische Beispieie für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Anlagerungsprodukte von 15 bis 60 Mol Ethylenoxid an Ricinusöl und/oder gehärtetes Ricinusöl;
(5) Polyol- und insbesondere Polyglycerinester wie z.B. Polyglycerinpolyricinoleat
(6) Anlagerungsprodukte von 2 bis 15 Mol Ethylenoxid an Ricinusöl und/oder gehärtetes-Ricinusöl;
(7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(8) Trialkylphosphate sowie Mono-, Di- und/oder Tri-PEG-alkylphosphate;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß **DE-PS1165574** und/oder Mischester von Fettsäuren mit 6 bis 22 Kohlenstoffatomen, Methylglucose und Polyolen, vorzugsweise Glycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Als weitere Emulgatoren kommen ferner auch kationische Tenside, vorzugsweise Esterquats und insbesondere solche vom Typ der methylquaternierten Fettsäuretriethanolaminsalze in Frage.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Geeignete **Verdickungsmittel** sind beispielsweise Polysaccharide, insbesondere Xanthan-Gum, Guar-Guar, Agar-Agar, Alginate und Tylosen, Carboxy-methylcellulose und Hydroxyethylcellulose, ferner höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Periglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Zur Herstellung verschiedener O/W-Selbstbräunungslotionen wurden die Einsatzstoffe gemäß Tabelle 1 nach dem PIT-Verfahren vermischt. Die erfindungsgemäßen Zubereitungen 1 bis 6 sowie die beiden Vergleichslotionen V1 und V2 wurden anschließend 2 Tage bei 35°C gelagert. Die Viskosität wurde nach der Brookfield-Methode in einem RVT-Viskosimeter (5 Upm, Spindel 4) bestimmt, die Beurteilung der Viskosität erfolgte visuell. Dabei bedeutet (+) eine stabile, homogene Emulsion und (-) Entmischung. Die Ergebnisse sind in Tabelle 1 zusammengefaßt.

**Tabelle 1**

| **Lagerstabilität von O/W-Selbstbräunungslotionen** | | | | | | | | |
|---|---|---|---|---|---|---|---|---|
| **Komponenten** | **1** | **2** | **3** | **4** | **5** | **6** | **V1** | **V2** |
| Eumulgin® VL 75 ¹⁾ | 5,0 | 5,0 | 5,0 | 5,0 | 5,0 | 5,0 | - | - |
| Montanov® 68 ²⁾ | - | - | - | - | - - | - | 5,0 | 5,0 |
| Ceteareth-20 | - | - | - | - | 2,0 | - | - | 2,0 |
| Glyceryl Stearate | 5,0 | 5,0 | 5,0 | 5,0 | 5,0 | 5,0 | 5,0 | 5,0 |
| Dihydroxyaceton | 2,0 | -2,0 | 2,0 | 2,0 | 2,0 | 2,0 | 2,0 | 2,0 |
| Titandioxid | - | 1,0 | - | 0,5 | - | 1,0 | - | - |
| Tocopherol Acetate | - | - | 1,0 | 0.5 | - | 1,0 | - - | - |
| Coco Glycerides | 10,0 | 10,0 | 10,0 | 10,0 | 10,0 | 10,0 | 10,0 | 10,0 |
| Oleyl Oleate | 7,0 | 7,0 | 7,0 | -7,0 | 7,0 | 7,0 | 7,0 | 7,0 |
| Mandelöl | 3,0 | 3,0 | 3,0 | 3,0 | 3,0 | 3,0 | 3,0 | 3,0 |
| Wasser | ad 100 | | | | | | | |
| ***Lagerstabilität*** | | | | | | | | |
| ***- Viskosität (sofort, 20°C) [mPas]*** | 7000 | 8000 | 7000 | 7000 | 5000 | 7000 | 7000 | 6000 |
| ***- Viskosität (nach 2 d, 35°C) [mPas]*** | 6900 | 7800 | 6900 | 7000 | 4800 | 6900 | 3500 | 4200 |
| ***- Stabilität (nach 2 d, 35°C)*** | + | + | + | + | + | + | - | - |

| | | | | | | | | |
|---|---|---|---|---|---|---|---|---|
| **Legende:** 1) Polyglyceryl-2 Dipolyhydroxystearate (and) Lauryl Glucoside (Henkel) | | | | | | | | |
| 2) Cetearyl Glucoside (and) Cetearyl Alcohol (SEPPIC) | | | | | | | | |

## Patentansprüche

1. Kosmetische Zubereitungen mit einem Gehalt an Dihydroxyaceton und Alkyl- und/oder Alkenyloligoglykoside, **dadurch gekennzeichnet, daß** sie als Co-Emulgatoren weiterhin Polyolpoly-12-hydroxystearate enthalten.

2. Zubereitungen nach Anspruch 1, **dadurch gekennzeichnet, daß** sie Alkyl- und/oder Alkenyloligoglykoside der Formel **(I)** enthalten,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Zubereitungen nach den Ansprüchen 1 und 2, **dadurch gekennzeichnet, daß** sie Polyglycerinpoly-12-hydroxysterate enthalten

4. Zubereitungen nach mindestens einem der Ansprüche 1 bis 3, **dadurch gekennzeichnet, dass** die Alkyl- und/oder Alkenyloligoglykoside und die Polyolpoly-12-hydroxystearate im Gewichtsverhältnis 90 : 10 bis 10 : 90 enthalten sind.

5. Zubereitungen nach den Ansprüchen 1 bis 4, **dadurch gekennzeichnet, daß** sie Ölkörper enthalten, die ausgewählt sind aus der Gruppe, die gebildet wird von Guerbetalkoholen auf Basis von C₆-C₁₈-Fettalkoholen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estem von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estem von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethern, Siliconölen und/oder aliphatischen bzw. naphthenischen Kohlenwasserstoffen.

6. Zubereitungen nach mindestens einem der Ansprüche 1 bis 5, enthaltend
(a) 0,1 bis 30 Gew.-% Alkyl- und/oder Alkenyloligoglykoside,
(b) 0,1 bis 30 Gew.-% Polyolpoly-12-hydroxystearate,
(c) 0,01 bis 6 Gew.-% Dihydroxyaceton und
(d) 1 bis 90 Gew.-% Ölkörper,
mit der Maßgabe, daß sich die Mengenangaben gegebenenfalls mit Wasser und üblichen Hilfs- und Zusatzstoffen zu 100 Gew.-% ergänzen.

7. Verwendung von Mischungen aus
(a) Alkyl- und/oder Alkenyloligoglykosiden und
(b) Polyolpoly-12-hydroxystearaten
als Emulgatoren für Dihydroxyaceton zur Herstellung von Selbstbräunungsmitteln.

## Claims

1. Cosmetic preparations containing dihydroxyacetone and alkyl and/or alkenyl oligoglycosides, **characterized in that** they additionally contain polyolpoly-12-hydroxystearates as co-emulsifiers.

2. Preparations as claimed in claim 1, **characterized in that** they contain alkyl and/or alkenyl oligoglycosides corresponding to formula (I):
**R**^{**1**}**O-[G]**_{**p**} **(I)**
where R¹ is an alkyl and/or alkenyl radical containing 4 to 22 carbon atoms, G is a sugar unit containing 5 or 6 carbon atoms and p is a number of 1 to 10.

3. Preparations as claimed in claims 1 and/or 2, **characterized in that** they contain polyglycerol poly-12-polyhydroxystearates.

4. Preparations as claimed in claims 1 to 3, **characterized in that** they contain components (a) and (b) in a ratio by weight of 90:10 to 10:90.

5. Preparations as claimed in at least one of claims 1 to 4, **characterized in that** they contain oils selected from the group consisting of Guerbet alcohols based on C₆₋₁₈ fatty alcohols, esters of linear C₆₋₂₂ fatty acids with linear C₆₋₂₂ fatty alcohols, esters of branched C₆₋₁₃ carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/ triglyceride mixtures based on C₆₋₁₈ fatty acids, esters of C₆₋₂₂ fatty alcohols and/or Guerbet alcohols with aromatic carboxylic acids, vegetable oils, branched primary alcohols, substituted cycloyhexanes, linear C₆₋₂₂ fatty alcohol carbonates, Guerbet carbonates, esters of benzoic acid with linear and/or branched C₆₋₂₂ alcohols, dialkyl ethers, silicone oils and/or aliphatic or naphthenic hydrocarbons.

6. Preparations as claimed in at least one of claims 1 to 5 containing
(a) 0.1 to 30% by weight alkyl and/or alkenyl oligoglycosides,
(b) 0.1 to 30% by weight polyol poly-12-hydroxystearates,
(c) 0.01 to 6% by weight dihydroxyacetone and
(d) 1 to 90% by weight oils,
with the proviso that the quantities shown add up to 100% by weight, optionally with water and typical auxiliaries and additives.

7. The use of mixtures of
(a) alkyl and/or alkenyl oligoglycosides and
(b) polyol poly-12-hydroxystearates
as emulsifiers for dihydroxyacetate for the production of self-tanning preparations.

## Revendications

1. Préparations cosmétiques contenant de la dihydroxyacétone et un alkyl- et/ou alkényleoligoglucoside,
**caractérisées en ce qu'**
elles renferment comme agents co-émulsionnants en outre des poly-12-hydroxystéarates de polyols.

2. Préparations selon la revendication 1,
**caractérisées en ce qu'**
elles renferment des alkyl- et/ou alkényloligoglycosides de formule (I) :
R¹O - [G]ₚ (I)
dans laquelle R¹ représente un reste alkyle et/ou alkényle ayant de 4 à 22 atomes de carbone, G représente un reste de sucre ayant 5 ou 6 atomes de carbone, et p représente des nombres allant de 1 à 10.

3. Préparations selon les revendications 1 ou 2,
**caractérisées en ce qu'**
elles contiennent des poly-12-hydroxystéarates de polyglycérol.

4. Préparations selon les revendications 1 et 2,
**caractérisées en ce que**
les alkyl- et/ou alkényloligoglycosides et les poly-12-hydroxystéarates de polyols, sont présents dans un rapport en poids allant de 90 : 10 à 10 : 90.

5. Préparations selon les revendications 1 à 4,
**caractérisées en ce qu'**
elles renferment des composés huileux choisis dans le groupe formé des alcools de Guerbet à base d'alcools gras en C₆-C₁₈, des esters d'acides gras linéaires en C₆-C₂₂ avec des alcools gras en C₆-C₂₂ linéaires, des esters d'acides carboxyliques ramifiés en C₆-C₁₃ avec des alcools gras linéaires en C₆-C₂₂, des esters d'acides gras linéaires en C₆-C₂₂ avec des alcools ramifiés, des esters d'acides gras linéaires et/ou ramifiés avec des alcools plurifonctionnels et/ou des alcools de Guerbet, des triglycérides à base d'acides gras en C₆-C₁₀, des mélanges liquides de mono- /di- / triglycérides à base d'acide gras en C₆-C₁₈, es esters d'alcools gras en C₆-C₂₂ et/ou d'alcools de Guerbet avec des acides carboxyliques aromatiques, des huiles végétales, des alcools primaires ramifiés, des cyclohexanes substitués, des carbonates d'alcools gras en C₆-C₂₂, des carbonates de Guerbet, des esters d'acide benzoïque avec des alcools linéaires et/ou ramifiés en C₆-C₂₂, des éthers dialkyliques, des huiles de silicone, et/ou des hydrocarbures alyphatiques ou naphténiques.

6. Préparations selon au moins une des revendications 1 à 5,
contenant :
(a) de 0,1 à 30 % en poids d'alkyl- et/ou d'alkényloligoglycosides ;
(b) de 0,1 à 30 % en poids de poly 12-hydroxystéarates de polyol ;
(c) de 0,01 à 6 % en poids de dihydroxyacétone ; et
(d) de 1 à 90 % en poids de composé huileux
avec la précision que les données en quantités se complètent avec de l'eu et des adjuvants et additifs usuels à 100 % en poids.

7. Utilisation de mélanges à base de :
(a) alkyl- et/ou alkényloligoglycosides ; et
(b) poly-12-hydroxystéarates de polyol
comme agents émulsionnants pour la dihydroxyacétone en vue de la préparation de produits d'autobronzage.
